# EUROPEAN PATENT APPLICATION

(11) **EP 1 922 975 A1**
(43) Date of publication of application: **21.05.2008**
(21) Application number: 06746739.9
(22) Date of filing: 23.05.2006
(51) Int. Cl.: A61B 1/00, A61B 19/00

(54) **MEDICAL DEVICE**

(30) Priority: 06.09.2005 JP 2005257967
(71) Applicant: OLYMPUS MEDICAL SYSTEMS CORP., Shibuya-ku Tokyo 151-0072 (JP)
(72) Inventor: KANAZAWA, Noriaki c/o Intellectual Property Support Department, 2-3 Kuboyama-cho, Hachioji-shi, Tokyo 192-8512 (JP)
(74) Representative: von Hellfeld, Axel
(86) International application number: PCT/JP2006/310255
(87) International publication number: WO 2007/029379

(57) **Abstract**

A medical device (10) includes a medical instrument main body (32), an elongated and flexible first flexible member (36), an elongated and flexible second flexible member (82), an arm (104), a first holding member (134), and a second holding member (134). The first flexible member extends from the medical instrument main body. The second flexible member extends from the medical instrument main body in a direction different from that of the first flexible member or from a different position. The arm turnably supports the medical instrument main body. The first holding member movably holds the first flexible member. The second holding member movably holds the second flexible member.

## Description

### Technical Field

This invention relates to a medical device for holding a medical instrument having a cable such as an endoscope or an electric scalpel.

### Background Art

For example, a medical holder is disclosed in Jpn. Pat. Appln. KOKAI Publication No. 2003-70803. The holder holds an endoscope by an arm, and movably supports a plurality of cables extending from the endoscope by the arm of the holder. When the endoscope is turned about the axis of an insertion section, the cables move together with the turning. This enables the endoscope to turn about the axis of the insertion section so that interference is prevented by the movement of the cables.

In Jpn. Pat. Appln. KOKAI Publication No. 9-75370, there are provided a helical cable fastener and a tube packing member for turnably holding a plurality of cables and a plurality of tubes. The cables and tubes are passed through the centers of the cable fastener and tube packing member such that these cables and tubes are held to be able to turn or rotate about their axes in a packed state.

Jpn. Pat. Appln. KOKAI Publication No. 2001-187066 has disclosed equipment for bundling and suspending cables of an endoscope across a drape. Here, a plurality of cables are packed by a band provided to hang from an arm, and held in a suspended state. Thus, the cables are held to be able to turn or rotate about their axes in a packed state.

The holders disclosed in Jpn. Pat. Appln. KOKAI Publication No. 2003-70803, Jpn. Pat. Appln. KOKAI Publication No. 9-75370 and Jpn. Pat. Appln. KOKAI Publication No. 2001-187066 hold a plurality of cables all together by a holding member, so that when each cables is to be moved, the movement of other cables interferes with this cable, that is, free movement of a medical instrument is inhibited.

Particularly when there are flexible members extending from the medical instrument in different directions or extending from different positions of the medical instrument, the flexible members tend to independently move as the medical instrument is turned or rotated about the axes of the flexible members. Thus, the flexible members of the medical instrument form a looped state at some position and twine around the medical instrument, so that it may be difficult to turn the medical instrument about its axis. Therefore, it may be difficult to turn the medical instrument itself about the axes of the flexible members.

### Disclosure of Invention

This invention has been made to solve such a problem, and is directed to providing a medical device capable of maintaining a condition where a medical instrument having a plurality of flexible members extending in different directions or extending from different positions is easily turned or rotated about the axes of the flexible members.

A medical device according to the present invention includes a medical instrument main body, an elongated and flexible first flexible member, an elongated and flexible second flexible member, an arm, a first holding member, and a second holding member. The first flexible member extends from the medical instrument main body. The second flexible member extends from the medical instrument main body in a direction different from that of the first flexible member or from a different position. The arm turnably supports the medical instrument main body. The first holding member movably holds the first flexible member. The second holding member movably holds the second flexible member.

A medical device according to the present invention includes an arm, a medical instrument main body, an elongated and flexible first flexible member, an elongated and flexible second flexible member and at least one support portion. The medical instrument main body is turnably supported by the arm. The first flexible member is extending from the medical instrument main body. The second flexible member is extending from the medical instrument main body in a direction different from that of the first flexible member or from a different position. The support portion movably supports the first flexible member and the second flexible member.

### Brief Description of Drawings

FIG. 1 is a schematic diagram showing the configuration of a medical device according to a first embodiment of the present invention;
FIG. 2 is a schematic diagram showing a detailed configuration of the medical device according to the first embodiment;
FIG. 3A is a schematic elevation view showing a first support portion in the medical device according to the first embodiment;
FIG. 3B is a schematic sectional view of the first support portion in the medical device according to the first embodiment along the 3B-3B line in FIG. 3A;
FIG. 3C is a schematic diagram showing the observation of the first support portion in the medical device according to the first embodiment from the direction of an arrow 3C in FIG. 3A;
FIG. 3D is a schematic diagram showing the inverted arrangement of the first support portion in the medical device according to the first embodiment shown in FIG. 3A;
FIG. 4 is a schematic diagram showing the configuration of a medical device according to a second embodiment of the present invention;
FIG. 5A is a schematic elevation view showing a first support portion in the medical device according to the second embodiment;
FIG. 5B is a schematic sectional view of the first support portion in the medical device according to the second embodiment along the 5B-5B line in FIG. 5A;
FIG. 5C is a schematic diagram showing the observation of the first support portion in the medical device according to the second embodiment from the direction of an arrow 5C in FIG. 5A;
FIG. 6A is a schematic diagram showing the configuration of a medical device according to a third embodiment of the present invention;
FIG. 6B is a schematic diagram showing the cross section of a first tube in the medical device according to the third embodiment of the present invention;
FIG. 7A is a schematic elevation view showing a third support portion in the medical device according to the third embodiment;
FIG. 7B is a schematic sectional view of the third support portion in the medical device according to the third embodiment along the 7B-7B line in FIG. 7A;
FIG. 7C is a schematic diagram showing the observation of the third support portion in the medical device according to the third embodiment from the direction of an arrow 7C in FIG. 7A;
FIG. 7D is a schematic diagram showing the inverted arrangement of the third support portion in the medical device according to the third embodiment shown in FIG. 7A;
FIG. 8 is a schematic diagram showing the configuration of a medical device according to a fourth embodiment of the present invention;
FIG. 9A is a schematic elevation view showing a first support portion in the medical device according to the fourth embodiment;
FIG. 9B is a schematic diagram showing the observation of the first support portion in the medical device according to the fourth embodiment from the direction of an arrow 9C in FIG. 9A;
FIG. 9C is a schematic diagram showing the inverted arrangement of the first support portion in the medical device according to the fourth embodiment shown in FIG. 9A; and
FIG. 10 is a schematic diagram showing a modification of the configuration of the medical device according to the fourth embodiment of the present invention.

### Best Mode for Carrying Out the Invention

The best mode for carrying out this invention will hereinafter be described with reference to the drawings.

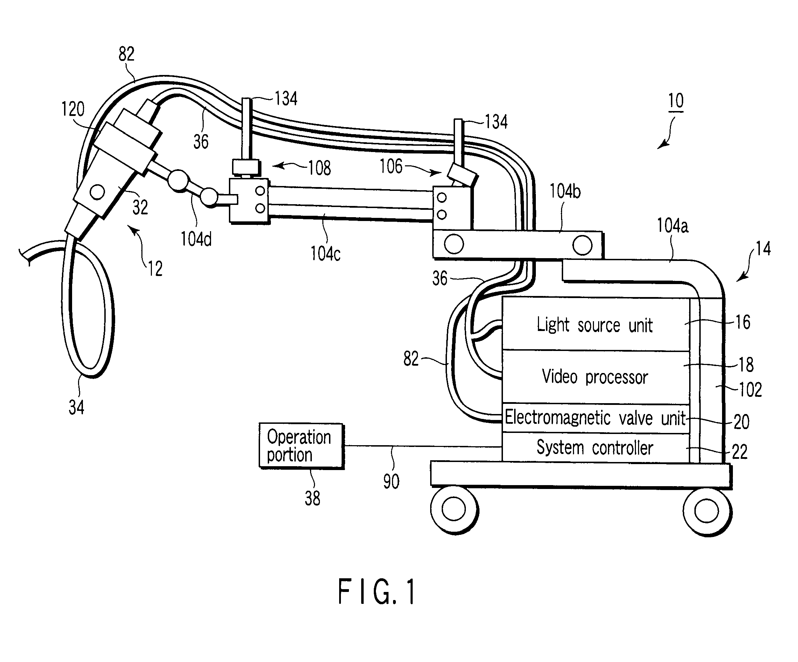

A first embodiment is explained with FIGS. 1 to 3D.

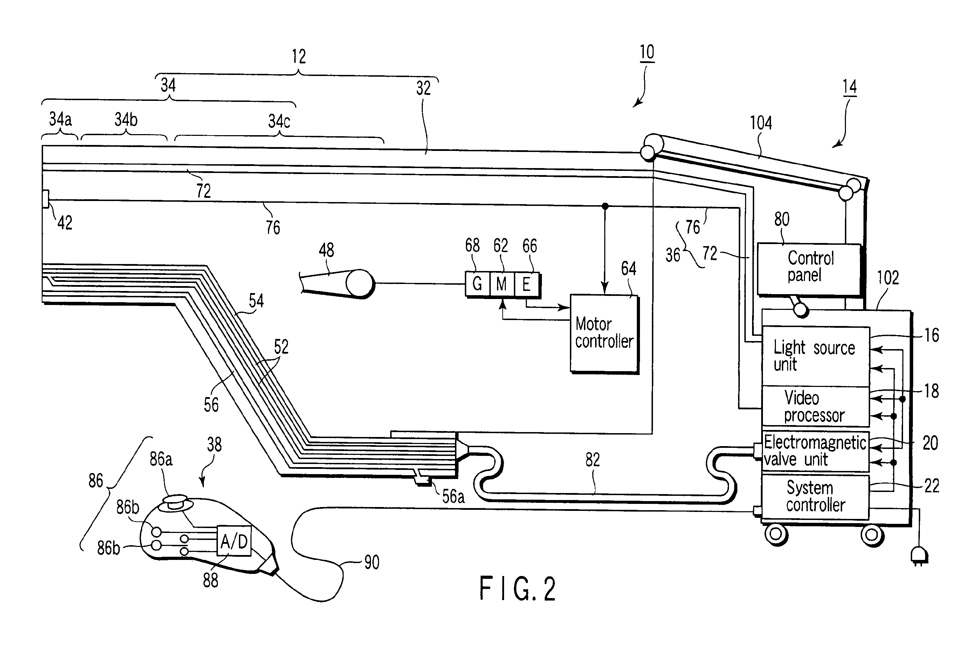

As shown in FIGS. 1 and 2, a medical device 10 according to this embodiment includes an electric bending type endoscope (medical instrument) 12, a supporter 14, a light source unit 16, a video processor 18, an electromagnetic valve unit 20 and a system controller 22. The electric bending type endoscope 12 has functions for observing and operating on the inside of a body cavity. The supporter 14 supports the electric bending type endoscope 12 movably within a predetermined range. The light source unit 16 supplies an illumination light flux to be emitted from the front surface of the distal end of an insertion section 34 described later. The video processor 18 receives a video signal from an imaging unit 42 described later, and then subjects the video signal to predetermined signal processing. The electromagnetic valve unit 20 controls air supply/water supply and suction operations via, for example, an air supply/water supply conduit 52 and a suction conduit 54 described later which are provided inside the insertion section 34. The system controller 22 is electrically connected to the light source unit 16, the video processor 18 and the electromagnetic valve unit 20. Thus, the system controller 22 controls the driving of a bending drive mechanism 44 described later, and can collectively control the light source unit 16, the video processor 18 and the electromagnetic valve unit 20.

As shown in FIG. 2, the endoscope 12 integrally includes a base portion (medical instrument main body) 32 which has, for example, a substantially cylindrical shape or a substantially columnar shape, the elongated insertion section 34 extending from one side surface of the base portion 32, and an elongated universal cable (first flexible member) 36 extending from the other side surface of the base portion 32. The insertion section 34 and the universal cable 36 are disposed on the same axis with respect to the base portion 32. Both the insertion section 34 and the universal cable 36 are flexible. The end of the universal cable 36 is optically connected to the light source unit 16, and electrically connected to the video processor 18. The endoscope 12 additionally includes an operation portion 38 for bending a bending portion 34b described later and for performing air supply/water supply and suction. The operation portion 38 is electrically connected to the system controller 22.

The insertion section 34 includes a distal end rigid portion 34a formed on the most distal side of the insertion section 34, the bending portion 34b continuously provided on the proximal side of the distal end rigid portion 34a, and a flexible tube portion 34c which is continuously provided on the proximal side of the bending portion 34b and which is formed into an elongated shape. The distal end rigid portion 34a has the built-in imaging unit 42 composed of an imaging optical system (not shown), an image pickup device such as a CCD, etc. The bending portion 34b is configured to bend vertically and horizontally by the driving control of the later-described bending drive mechanism 44 controlled in accordance with a bending instruction provided by the operation portion 38.

The base portion 32 has the built-in bending drive mechanism 44 for bending the bending portion 34b. The insertion section 34 extending from the base portion 32 is inserted into a passage in the body cavity and is therefore flexible. To the end of the universal cable 36 extending from the other side of the base portion 32, the light source unit 16 is optically connected and the video processor 18 is electrically connected.

Furthermore, an angle wire 48 driven in response to the driving force from the bending drive mechanism 44 is inserted through the insertion section 34. Although not shown, the angle wire 48 is connected to the distal side of the bending portion 34b. Therefore, if the angle wire 48 is driven in response to the driving force from the bending drive mechanism 44 of the base portion 32, the bending portion 34b bends vertically and horizontally.

The air supply/water supply conduit 52 and the suction conduit 54 are inserted through the insertion section 34.
An air supply/water supply opening is made at the distal end of the air supply/water supply conduit 52, and a suction opening is made at the distal end of the suction conduit 54. An air supply/water supply opening is made in the base portion 32 at the proximal end of the air supply/water supply conduit 52, and a suction opening is made in the base portion 32 at the proximal end of the suction conduit 54. One end of a tube 82 described later is connected to the air supply/water supply opening at the proximal end of the air supply/water supply conduit 52 and the suction opening at the proximal end of the suction conduit 54. That is, one end of the tube 82 is connected to the base portion 32. Moreover, a forceps conduit 56 for inserting a medical instrument such as forceps is inserted through the insertion section 34. A forceps opening is made in the distal front surface of the forceps conduit 56. The proximal end of the forceps conduit 56 is inserted, at the proximal end of the insertion section 34, through a forceps insertion opening 56a formed in the vicinity of the base portion 32. Thus, the medical instrument such as the forceps inserted from the forceps insertion opening 56a can project from the distal front surface of the insertion section 34 through the forceps conduit 56.

The bending drive mechanism 44 is bending drive means composed of an electric motor 62 and various members formed to transmit and separate the driving force generated from the electric motor 62. The bending drive mechanism 44 includes the electric motor 62, a motor controller 64, an encoder 66 and a reduction gear 68.

The electric motor 62 generates driving force by rotation. The motor controller 64 performs overall control of the bending drive mechanism 44 including the electric motor 62. The encoder 66 converts the operating states such as the rotation speed and rotation amount of the drive shaft of the electric motor 62 into data. The reduction gear 68 reduces the rotational power of the drive shaft of the electric motor 62.

A light guide 72 is connected to the light source unit 16. The light guide 72 is provided to extend to the distal end of the insertion section 34 through the universal cable 36, the base portion 32 and the insertion section 34. Thus, the illumination light flux supplied from the light source unit 16 is emitted from the distal end of the insertion section 34 via the light guide 72.

A signal cable 76 for transmitting the video signal from the imaging unit 42 is connected to the video processor 18. The signal cable 76 extends from the imaging unit 42 at the distal end of the insertion section 34, and is connected to a predetermined terminal of the video processor 18 through the insertion section 34, the base portion 32 and the universal cable 36. Further, a control panel 80 is electrically connected to the video processor 18. The video signal output from the video processor 18 is transmitted to the control panel 80. In response to this, a predetermined endoscopic image is displayed using a display unit in the control panel 80. In addition to the display unit, the control panel 80 is provided with an operation unit on the display surface of the display unit. Thus, various operational instructions can be input from the operation unit.

A pair of tubes (second flexible member) 82 in communication with the air supply/water supply conduit 52 and the suction conduit 54 of the insertion section 34 is connected to the electromagnetic valve unit 20. That is, the electromagnetic valve unit 20 is in communication with the distal end of the insertion section 34 via the tube 82, the air supply/water supply conduit 52 and the suction conduit 54. Thus, when the electromagnetic valve unit 20 is driven to perform air supply/water supply operation, air/water can be supplied from the distal end of the insertion section 34 through the tube 82 and the air supply/water supply conduit 52 of the base portion 32 and the insertion section 34. Moreover, when the electromagnetic valve unit 20 is driven to perform suction operation, suction can be performed from the distal end of the insertion section 34 through the suction conduit 54 of the insertion section 34 and the base portion 32 and the tube 82. In addition, the tube 82 includes two tubes for air supply/water supply and for suction, and these tubes are described here on the assumption that they are bundled. Moreover, while the tube 82 is formed of a hollow flexible resin material, it is difficult for the universal cable 36 to be flexible as compared with the tube 82 including two bundled tubes because the light guide 72 and the signal cable 76 are provided in the universal cable 36. That is, the tube 82 is formed to have a torque transmission rate lower than that of the universal cable 36.

The operation portion 38 includes various operation members for generating a bending operation instruction signal, an air supply/water supply operation instruction signal and a suction operation instruction signal, and is configured separately from the base portion 32. The operation portion 38 includes various operation members 86 and an analog-to-digital converter 88. The various operation members 86 include an operation stick 86a for providing the bending operation instruction, and an operation button 86b for providing the air supply/water supply operation instruction signal and the suction operation instruction. The various operation members 86a and 86b are electrically connected to the analog-to-digital converter 88. Thus, the analog-to-digital converter 88 performs analog-to-digital conversion processing for receiving electric signals generated from the various operation members 86a, 86b and converting the electric signals into predetermined operation instruction signals.

The operation portion 38 is electrically connected to the system controller 22 by an electric cable 90. Thus, the various operation instruction signals generated by the analog-to-digital converter 88 when the various operation members of the operation portion 38 are operated are transmitted to the system controller 22 via the electric cable 90. Moreover, the light source unit 16, the video processor 18, the electromagnetic valve unit 20 and the control panel 80 are electrically connected to the system controller 22. Thus, on receipt of the various operation signals from the operation portion 38, the system controller 22 properly transmits control signals for performing control corresponding to the instruction signals to various instruments. Moreover, on receipt of the various operation instruction signals from the operation unit of the control panel 80, the system controller 22 properly transmits control signals for performing control corresponding to the instruction signals to various instruments.

The supporter 14 includes a supporter base 102, an arm 104 and first and second support portions 106, 108. The supporter base 102 is, for example, a cart provided with casters. The light source unit 16, the video processor 18, the electromagnetic valve unit 20, the system controller 22 and the control panel 80 are housed in the supporter base 102, and the supporter base 102 is movable on the floor with these units mounted thereon. The arm 104 supports the endoscope 12 and moves the endoscope 12 within a predetermined range. The first and second support portions 106, 108 are provided in, for example, the arm 104 in this embodiment. The first and second support portions 106, 108 support the universal cable 36 extending from the base portion 32 of the endoscope 12, and the tube 82 extending from the proximal end of the insertion section 34 of the endoscope 12, respectively. The first support portion 106 supports the universal cable 36 and the tube 82, and the second support portion 108 also supports the universal cable 36 and the tube 82.

As shown in FIG. 1, the arm 104 is supported by the supporter base 102. The arm 104 includes first to fourth arms 104a, 104b, 104c, 104d. One end of the first arm 104a is fixed to the supporter base 102. One end of the second arm 104b is horizontally movably supported at the other end of the first arm 104a by a vertically extending pin (not shown). That is, the second arm 104b is an arm for horizontally moving the endoscope 12. In addition, an unshown electromagnetic brake, for example, is provided around the pin which supports the other end of the first arm 104a and the one end of the second arm 104b. Therefore, the second arm 104b can be disposed at a predetermined position with respect to the first arm 104a within a predetermined turning range.

One end of the third arm 104c is vertically movably supported at the other end of the second arm 104b by a horizontally extending pin (not shown). That is, the third arm 104c is an arm for vertically moving the endoscope 12. In addition, an unshown electromagnetic brake, for example, is provided around the pin which supports the other end of the second arm 104b and the one end of the third arm 104c. Therefore, the third arm 104c can be disposed at a predetermined position with respect to the second arm 104b within a predetermined turning range.

One end of the fourth arm 104d is supported at the other end of the third arm 104c. An endoscope holding portion 120 is provided at the other end of the fourth arm 104d. The fourth arm 104d may obliquely hold the insertion section 34 of the endoscope 12, and can therefore tilt with one or a plurality of joints. Moreover, as the joints may be subjected to great force, it is preferable that an electromagnetic brake is provided in each joint. Thus, the endoscope 12 can be fixed at a desired angle within a predetermined range. Moreover, the base portion 32 of the endoscope 12 is supported rotatably about the axis of the insertion section 34 and the universal cable 36 by the endoscope holding portion 120.

For example, the upper surface of the one end of the third arm 104c is formed into a flat surface. The first support portion 106 is fixed to this upper surface of the one end of the third arm 104c. Moreover, for example, the upper surface of the other end of the third arm 104c is formed into a flat surface. The second support portion 108 is fixed to this upper surface of the other end of the third arm 104c. Here, the first support portion 106 is representatively explained on the assumption that the first support portion 106 and the second support portion 108 have the same configuration.

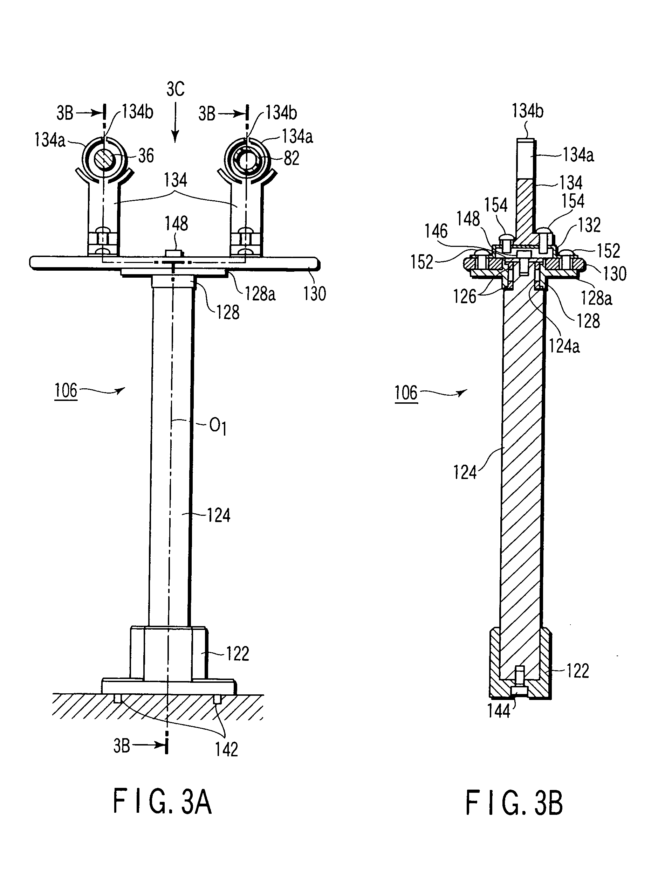

As shown in FIGS. 3A and 3B, the first support portion 106 includes a base portion 122, a shaft 124, bearings 126, a cylindrical member 128, a table 130, a holder 132 and bundle retainers 134.

As shown in FIG. 3A, the base portion 122 is fixed to the upper flat surface of the one end of the third arm 104c by screws 142. As shown in FIG. 3B, the base portion 122 is formed into a cylindrical shape with a bottom, and is fixed to the proximal end (here, lower end) of the shaft 124 by a screw 144.

At the distal end (here, upper end) of the shaft 124, a small diameter portion 124a smaller in diameter than the proximal end thereof is formed. Vertically aligned two bearings (first rotation axis O₁) 126 are fixed to the outer periphery of the small diameter portion 124a by a pin 148 via a presser plate 146. The cylindrical member 128 having a flange portion 128a is supported on the outer peripheries of the bearings 126. A table 130 substantially in the shape of a rectangular board is fixed to the upper side of the flange portion 128a of the cylindrical member 128 by screws 152. Thus, the table 130 is integrated with the cylindrical member 128, and can rotate with respect to the shaft 124 about the axis thereof. Two holders 132 are fixed to the table 130 by the above-mentioned screws 152. The proximal ends (here, lower ends) of the bundle retainers 134 are fixed to these holders 132 by screws 154.

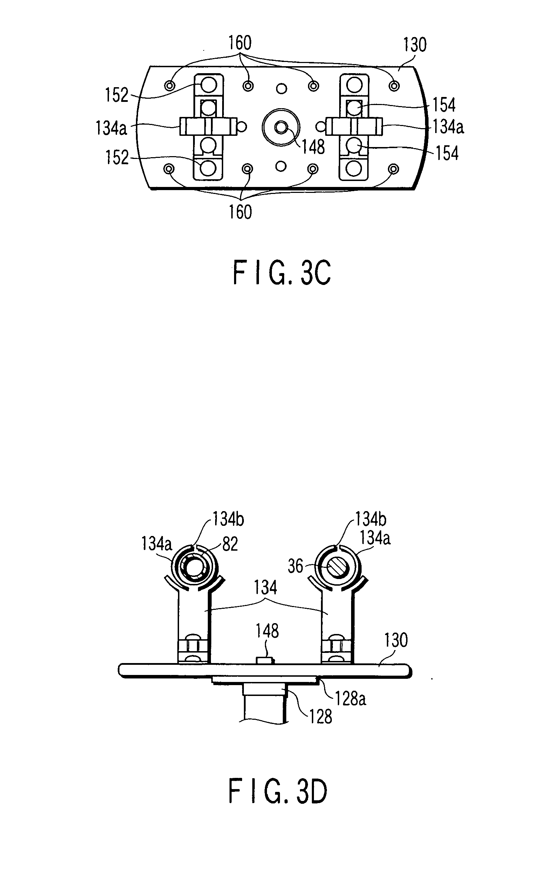

In addition, FIG. 3A shows the two holders 132 and two bundle retainers 134 disposed at positions symmetrical with respect to the central axis of the shaft 124, but it is also preferable that one of the holders 132 and one of the bundle retainers 134 are disposed at positions indicated by the reference number 160 shown in FIG. 3C. That is, the distance between a pair of bundle retainers 134 can be suitably changed. Further, more bundle retainers 134 can be disposed at the positions indicated by the reference number 160. That is, a total of six bundle retainers 134 can be arranged in the table 130 shown in FIG. 3C.

A C-ring portion (holding portion) 134a is formed at the distal end (here, upper end) of the bundle retainer 134. Therefore, if the C-ring portion 134a is elastically deformed, the universal cable 36 and the tube 82 can be placed from an opening 134b between the ends of the C-ring portion 134a. Here, the opening 134b is formed at the top end, but it is also preferable that the opening 134b is formed in the side portion of the C-ring portion 134a.

Here, for example, the universal cable 36 is disposed in the C-ring portion 134a of the one bundle retainer (first holding member) 134 by the elastic deformation of the opening 134b. For example, the tube 82 is disposed in the C-ring portion 134a of the other bundle retainer (second holding member) 134 by the elastic deformation of the opening 134b. Owing to the clearance between the universal cable 36 and the C-ring portion 134a and the clearance between the tube 82 and the C-ring portion 134a, these C-ring portions 134a allow the axial movement of the universal cable 36 and the tube 82 and also allow turning/rotation about their axes.

Incidentally, the second support portion 108 has the same configuration as that of the first support portion 106 as described above. Then, the universal cable 36 is disposed in a C-ring portion 134a of one bundle retainer (first holding member) 134 of the second support portion 108 by the elastic deformation of an opening 134b. Further, the tube 82 is disposed in a C-ring portion 134a of the other bundle retainer (second holding member) 134 by the elastic deformation of an opening 134b. That is, the universal cable 36 is disposed in the C-ring portions 134a of the bundle retainers (first holding members) 134 on one side of the first and second support portions 106, 108, and the tube 82 is disposed in the C-ring portions 134a of the bundle retainers (second holding members) 134 on the other side of the first and second support portions 106, 108.

Next, the effects of the medical device 10 according to this embodiment will be described.

The distal end of the insertion section 34 of the endoscope 12 shown in FIG. 2 is introduced into a desired position of, for example, a passage in a body cavity. At this point, instead of operating the operation portion 38 to bend the bending portion 34b, an operator may grip the insertion section 34 to insert the insertion section 34 while turning or rotating the insertion section 34 about its axis, as one technique to facilitate the insertion. When the insertion section 34 is turned in this manner, the turning force is transmitted to the base portion 32. The base portion 32 held by the endoscope holding portion 120 also turns along with the turning of the insertion section 34. Thus, the turning force of the insertion section 34 is further transmitted from the base portion 32 to the universal cable 36 and the tube 82.

Here, the end of the universal cable 36 is connected to the light source unit 16 and the video processor 18. Moreover, the end of the tube 82 is connected to the electromagnetic valve unit 20. Therefore, the turning amounts of the universal cable 36 and the tube 82 are limited. That is, the turning amounts of the base portion 32 and the insertion section 34 are also limited. Thus, when the operator grips the insertion section 34 to turn the insertion section 34, reaction force acts to return the turning. Meanwhile, the operator keeps exerting the turning force against the reaction force in order to hold the turned state of the insertion section 34.

In general, if a member such as the universal cable 36 or the tube 82 having proper flexibility and having one end fixed is turned in one direction, reaction force against the turning force is generated. If the universal cable 36 or the tube 82 is turned, a loop may be suddenly formed at some position to release the turning force (return the turned state to the original state). That is, the universal cable 36 or the tube 82 may unexpectedly move due to the turning reaction force. In such a case, this force may be transmitted from the universal cable 36 or the tube 82 to the insertion section 34 through the base portion 32.

Here, the universal cable 36 and the tube 82 are independently supported in the C-ring portions 134a of the bundle retainers 134 in both the first and second support portions 106, 108. Moreover, the universal cable 36 and the tube 82 are disposed with proper space therebetween in both the first and second support portions 106, 108. That is, the first and second support portions 106, 108 have proper space provided therebetween, and are arranged so that the universal cable 36 and the tube 82 do not cross each other between the first and second support portions 106, 108.

Thus, when the insertion section 34 is turned, the turning force is transmitted to the universal cable 36 and the tube 82 through the base portion 32. At this point, both the tables 130 of the first and second support portions 106, 108 freely turn about the axes of the shafts 124 to release as much force applied to the universal cable 36 and the tube 82 as possible. Therefore, the first and second support portions 106, 108 move to release the turning force applied to the universal cable 36 and the tube 82. Thus, even if a loop is to be suddenly formed in the universal cable 36 and the tube 82, the universal cable 36 and the tube 82 are untwisted by the first and second support portions 106, 108. That is, the force to return the rotation is prevented from being suddenly transmitted from the universal cable 36 and the tube 82 to the insertion section 34 through the base portion 32 as much as possible.

Furthermore, since the interference between the universal cable 36 and the tube 82 is prevented by the first and second support portions 106, 108, the tube 82, for example, is not subject to the turning force of the universal cable 36. Thus, even when the insertion section 34 is turned, a condition where the insertion section 34 is easily turned about its axis is maintained as much as possible because the interference between the universal cable 36 and the tube 82 is prevented.

As described above, the following effects are obtained according to this embodiment.

When the endoscope 12 is turned about the axis of the insertion section 34, it is possible to maintain a condition where the interference between the universal cable 36 and the tube 82 is prevented as much as possible because the positions of the universal cable 36 and the tube 82 are independently regulated by the first and second support portions 106, 108. Therefore, when the insertion section 34 of the endoscope 12 is turned, the universal cable 36 and the tube 82 are untwisted, and a condition where the insertion section 34 can is easily turned can be maintained as much as possible.

In addition, regarding the positions of the C-ring portions 134a of the first and second support portions 106, 108, it is preferable that the position of the C-ring portion 134a of the second support portions 108 is higher than the position of the C-ring portion 134a of the first support portion 106. Then, the universal cable 36 and the tube 82 extending from the base portion 32 of the endoscope 12 can be disposed from a high position to a low position, and it is possible to provide a smooth flow of the universal cable 36 and the tube 82 to the light source unit 16, the video processor 18 and the electromagnetic valve unit 20 that are arranged on a lower side.

Furthermore, as shown in FIG. 3D, it is also preferable that the universal cable 36 and the tube 82 disposed in the one bundle retainer 134 and the other are provided in a state horizontally inverted with respect to the state in FIG. 3A.

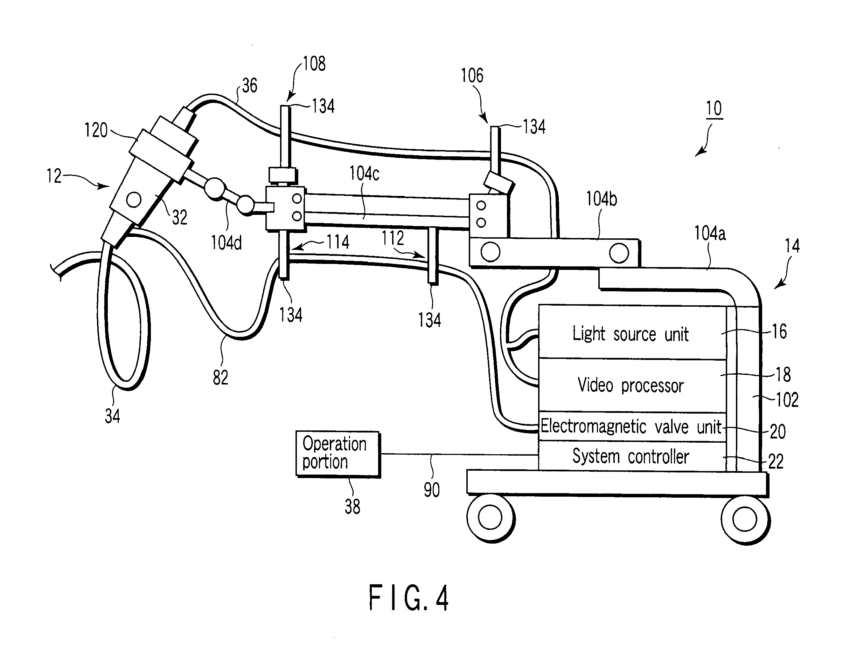

Next, a second embodiment is explained with FIGS. 4 to 5C. This embodiment is a modification of the first embodiment, so that the same reference numbers are assigned to the same members as the members described in the first embodiment or to members having the same function, and these members are omitting in detail.

As shown in FIG. 4, in this embodiment, a tube 82 extending from an endoscope 12 is disposed under an arm 104. Thus, bundle retainers 134 of first and second support portions 106, 108 disposed on the upper end faces of the one and the other ends of a third arm 104c only support a universal cable 36. That is, one bundle retainer 134 has only to be provided for each of the first and second support portions 106, 108.

Meanwhile, third and fourth support portions 112, 114 are further provided on a lower side located in the vicinity of one end of the third arm 104c and on the other end thereof. Bundle retainers 134 of these third and fourth support portions 112, 114 also support the tube 82 alone. That is, one bundle retainer 134 has only to be provided for each of the third and fourth support portions 112, 114.

The structures of the first to fourth support portions 106, 108, 112, 114 according the present embodiment will be described below. Here, the first support portion 106 is representatively explained as in the first embodiment.

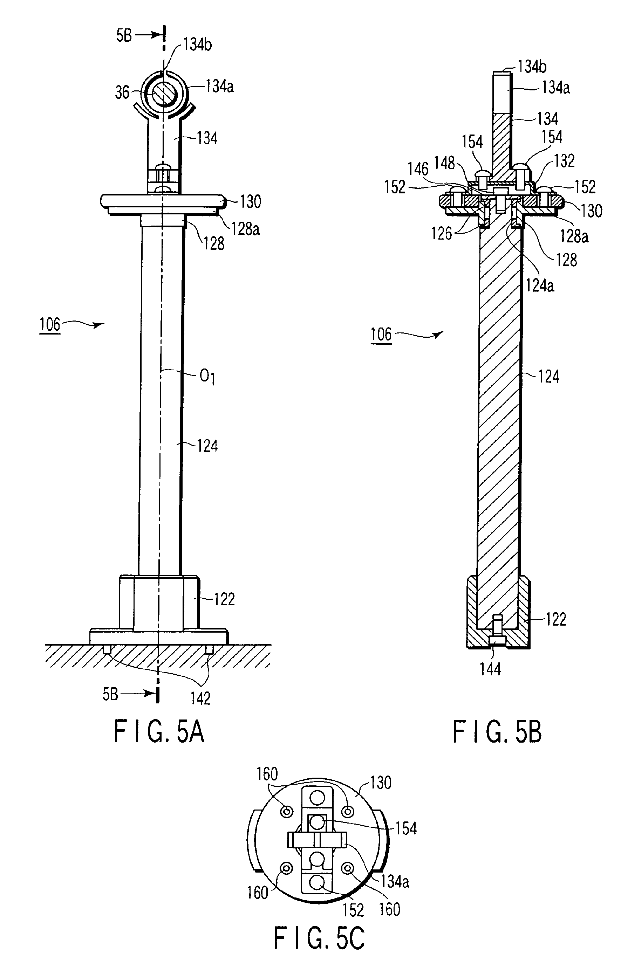

As shown in FIGS. 5A and 5B, the first support portion 106 includes a base portion 122, a shaft 124, bearings 126, a cylindrical member 128, a table 130, a holder 132 and bundle retainers 134, in the same manner as the first support portion 106 (see FIGS. 3A and 3B) described in the first embodiment.

As shown in FIG. 5C, the table 130 is substantially shaped like a disk, unlike the table 130 (see FIG. 3C) described in the first embodiment. That is, the table 130 turns or rotates with respect to the shaft 124, and the bundle retainer 134 turns or rotates about the axis of the shaft 124.

The shapes of the other members are the same as those in the first support portion 106 described in the first embodiment. Incidentally, in the third and fourth support portions 112, 114, the base portion 122 is disposed as an upper end, and the C-ring portion 134a of the bundle retainer 134 is disposed as a lower end. That is, the tube 82 can be placed from an opening 134b at the lowermost end of the C-ring portion 134a by the elastic deformation of the C-ring portion 134a.

In addition, regarding the positions of the C-ring portions 134a of the third and fourth support portions 112, 114, it is preferable that the position of the C-ring portion 134a of the fourth support portions 114 is higher than the position of the C-ring portion 134a of the third support portion 112. Then, the tube 82 extending from the base portion 32 of the endoscope 12 can be disposed from a high position to a low position, and it is possible to provide a smooth flow of the tube 82 to the electromagnetic valve unit 20 arranged on a lower side.

Furthermore, while the bundle retainers 134 of the first to fourth support portions 106, 108, 112, 114 are arranged on the same axis as the shafts 124 in this embodiment described above, it is also preferable that the bundle retainers 134 are arranged at position indicated by the reference number 160 shown in FIG. 5C.

The effects and advantages of the medical device 10 according to this embodiment are the same as the effects and advantages of the medical device 10 described in the first embodiment, and are therefore omitting a description thereof.

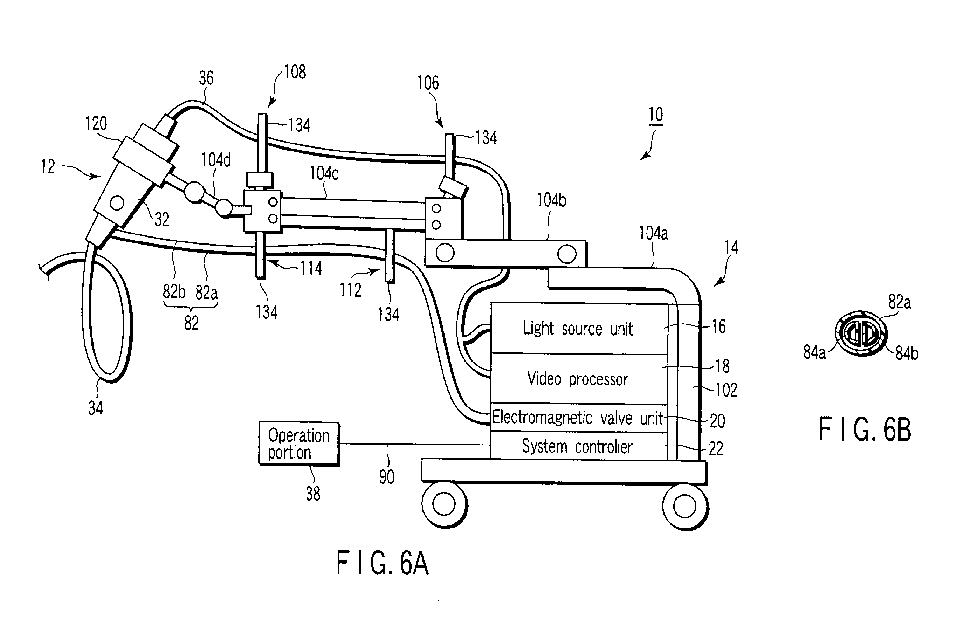

Next, a third embodiment is explained with FIGS. 6A to 7D. This embodiment is a modification of the first and second embodiments, so that the same reference numbers are assigned to the same members as the members described in the first and second embodiments or to members having the same function, and these members are omitting in detail.

In this embodiment, a tube 82 includes a first tube 82a for air supply and water supply and a second tube 82b for suction, as shown in FIG. 6A. That is, an explanation will be given here on the assumption that there are two tubes 82 in contrast with the first and second embodiments described above. In addition, as shown in FIG. 6B, the first tube 82a includes a lumen 84a for air supply and a lumen 84b for water supply.

Here, the structures of first and second support portions 106, 108 are similar to those described in the second embodiment, and are therefore omitting a description thereof. The structures of third and fourth support portions 112, 114 will hereinafter be explained. Here, the third support portion 112 is representatively explained.

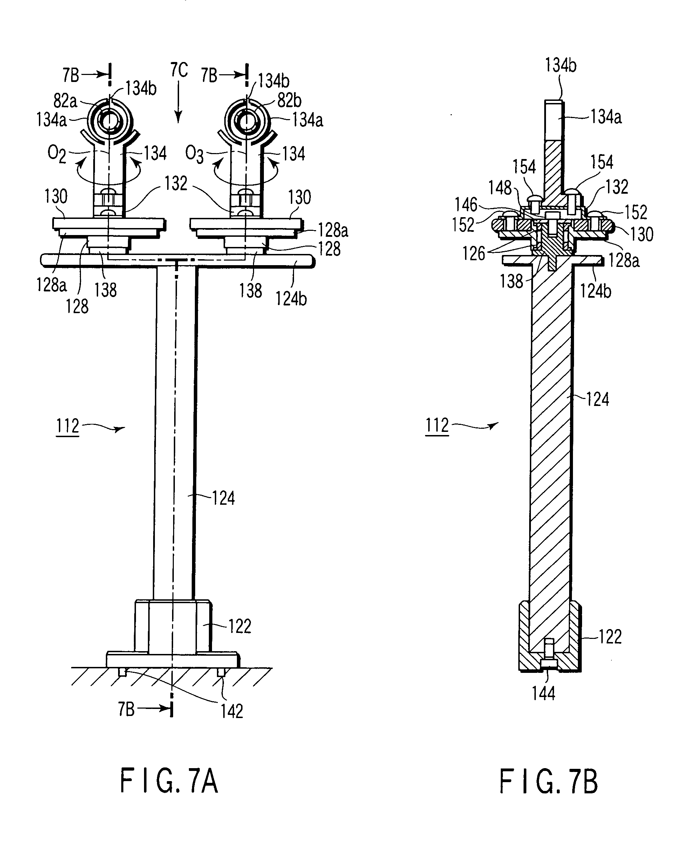

As shown in FIGS. 7A and 7B, the third support portion 112 includes a base portion 122, a shaft 124, pivot members 138, bearings 126, a cylindrical member 128, tables 130, holders 132 and bundle retainers 134.

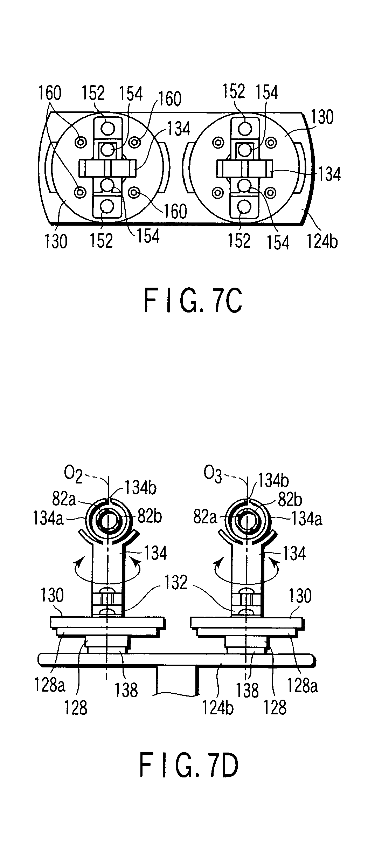

A flange portion 124b is formed at the distal end (upper end in FIGS. 7A and 7B) of the shaft 124. The flange portion 124b is shaped substantially like a rectangular board, as shown in FIG. 7C.

As shown in FIGS. 7A and 7B, a pair of pivot members 138 is fixed on the flange portion 124b at positions symmetrical with respect to the longitudinal axial direction of the shaft 124. A pair of bearings (second rotation axes O₂, O₃) 126 is fixed to the outer periphery of each of the pivot members 138 via a presser plate 146 by a pin 148. The cylindrical member 128 having a flange portion 128a is supported on the outer peripheries of these bearings 126. The configuration is similar to that of the first support portion 106 described in the first embodiment in other respects.

Therefore, in the third support portion 112, the adjacent bundle retainers 134 can turn (rotate) independently of each other. In the fourth support portion 114 having a similar configuration as well, adjacent bundle retainers 134 can turn (rotate) independently of each other.

Here, the first tube 82a is disposed in C-ring portions 134a of the bundle retainers (first holding members) 134 on one side of the third and fourth support portions 112, 114, and the second tube 82b is disposed in C-ring portions 134a of the bundle retainers (second holding members) 134 on the other side of the third and fourth support portions 112, 114.

Next, the effects of the medical device 10 according to this embodiment will be described. Here, the effects of the first and second tubes 82a, 82b disposed in the bundle retainers 134 of the third and fourth support portions 112, 114 in particular are mainly described. The effects of a universal cable 36 are similar to the effects described in the second embodiment, and are therefore omitting a detailed explanation thereof.

Both the first and second tubes 82a, 82b are supported by the C-ring portions 134a of the bundle retainers 134 of the third and fourth support portions 112, 114. Moreover, the first and second tubes 82a, 82b are disposed with proper space provided therebetween in both the third support portion 112 and the fourth support portion 114. That is, the third and fourth support portions 112, 114 have proper space provided therebetween, and are arranged so that the first tube 82a and the second tube 82b uncross each other between the third and fourth support portions 112, 114.

Thus, when an insertion section 34 is turned, the turning force is transmitted to the first and second tubes 82a, 82b through a base portion 32. At this point, both the tables 130 of the third and fourth support portions 112, 114 freely turn about the axes of the pivot members 138 on the flange portion 124b of the shaft 124 to release as much force applied to the universal cable 36 and the tube 82 as possible. Therefore, the third and fourth support portions 112, 114 move to release the turning force applied to the first and second tubes 82a, 82b. Thus, even if a loop is to be suddenly formed in the first and second tubes 82a, 82b, the unexpected movement of the first and second tubes 82a, 82b is prevented by the third and fourth support portions 112, 114. That is, the force to return the rotation is prevented from being suddenly transmitted from the first and second tubes 82a, 82b to the insertion section 34 through the base portion 32 as much as possible.

The effects of the medical device 10 according to this embodiment are the same as the effects of the medical device 10 described in the first embodiment, and are therefore omitting a description thereof.

In addition, it is also preferable that the third and fourth support portion 112, 114 described in the present embodiment is configured so that one bundle retainer 134 is provided in one table 130 as in the first support portion 106 (see FIGS. 5A and 5B) described in the second embodiment. In this case, the universal cable 36 and the first and second tubes 82a, 82b are provided in independent first support portions 106. Moreover, it is also preferable that the air supply/water supply tube which is the first tube 82a is supported after dividing the tube into two independent lumens for air supply/water supply and suction and providing corresponding bundle retainers 134.

Furthermore, as shown in FIG. 7D, it is also preferable that the first and second tubes 82a, 82 disposed in the one bundle retainer 134 and the other are provided in a state horizontally inverted with respect to the state in FIG. 7A.

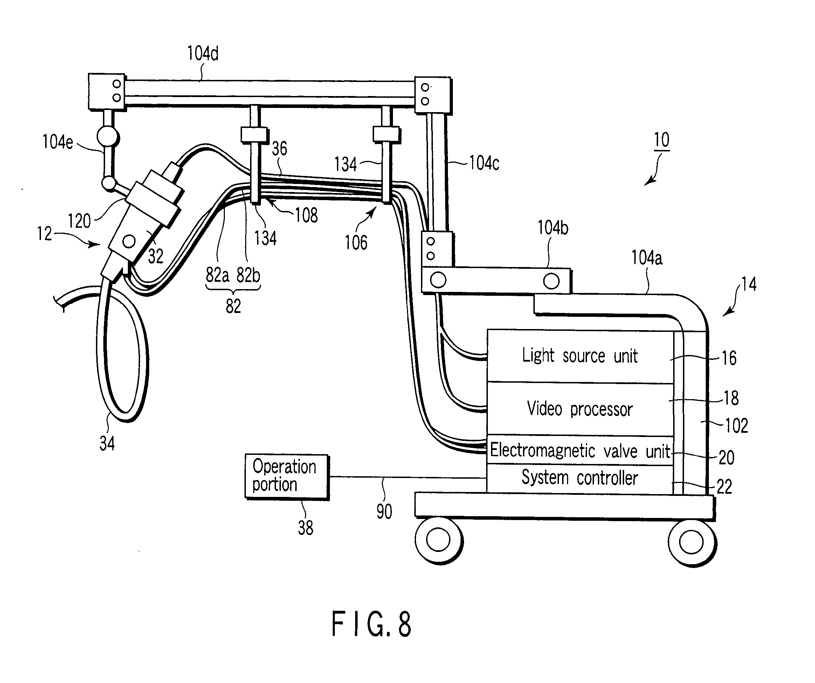

Next, a forth embodiment is explained with FIGS. 8 to 10. This embodiment is a modification of the first to third embodiments, so that the same reference numbers are assigned to the same members as the members described in the first to third embodiments or to members having the same function, and these members are omitting in detail.

As shown in FIG. 8, an arm 104 includes first to fifth arms 104a, 104b, 104c, 104d, 104e. One end (lower end) of the third arm 104c is provided to stand from the other end of the second arm 104b to the ceiling. One end of the fourth arm 104d is disposed at the other end (upper end) of the third arm 104c. The fourth arm 104d is disposed in parallel with the floor and the ceiling. One end (upper end) of the fifth arm 104e is connected to the other end of the fourth arm 104d. An endoscope holding portion 120 is disposed the other end of the fifth arm 104e. The fifth arm 104e may obliquely hold an insertion section 34 of an endoscope 12, and can therefore tilt. A base portion 32 of the endoscope 12 is rotatably supported by the endoscope holding portion 120.

In addition, it is preferable that the third arm 104c is vertically movably supported by a horizontally extending pin (not shown). It is also preferable that the fourth arm 104d is horizontally movably supported by a vertically extending pin (not shown).

The fourth arm 104d is provided with first and second support portions 106, 108. The structures of the first and second support portions 106, 108 according the present embodiment will be described below. Here, the first support portion 106 is representatively explained as in the first embodiment.

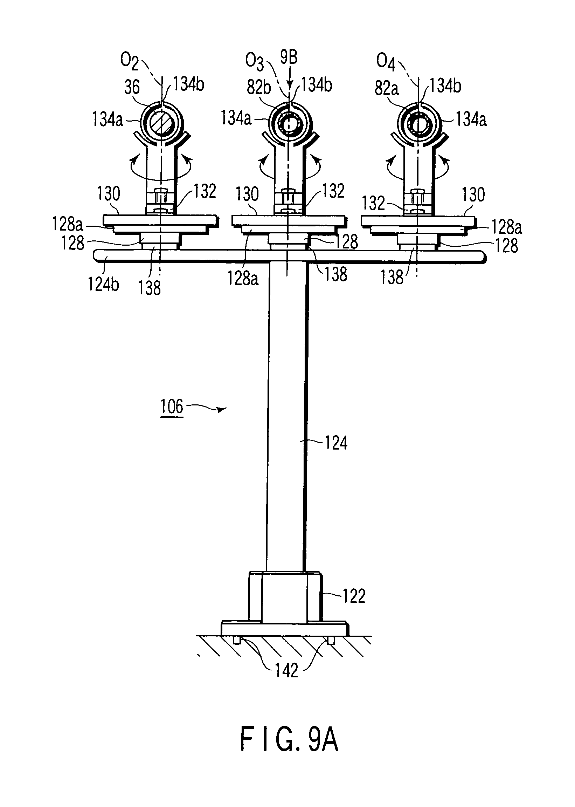

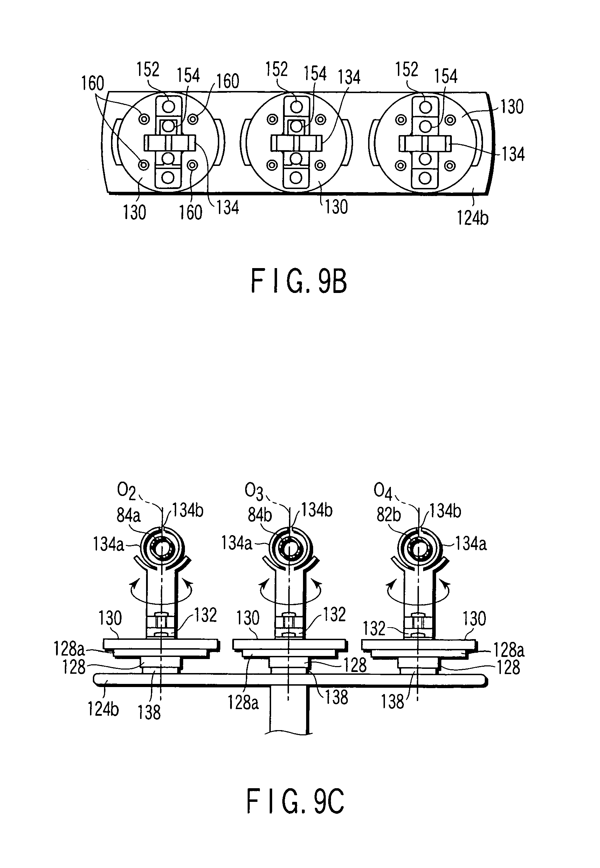

As shown in FIGS. 9A and 9B, the first support portion 106 includes a base portion 122, a shaft 124, pivot members 138, bearings (second rotation axes O₂, O₃, O₄) 126, cylindrical members 128, tables 130, holders 132 and bundle retainers 134, as in the third support portion 112 (see FIGS. 7A and 7B) described in the third embodiment.

On a flange portion 124b, one pivot member 138 is fixed on the same axis as the shaft 124, and a pair of pivot members 138 is fixed at positions symmetrical with respect to the longitudinal axial direction of the shaft 124. The configuration is similar to that of the third support portion 112 described in the third embodiment in other respects.

Therefore, in the first support portion 106, the adjacent bundle retainers 134 can turn (rotate) independently of each other. In the second support portion 108 having a similar configuration as well, adjacent bundle retainers 134 can turn (rotate) independently of each other.

Here, a universal cable 36 is disposed in C-ring portions 134a of the bundle retainers (first holding members) 134 on one side of the first and second support portions 106, 108. The first tube 82a is disposed in C-ring portions 134a of the bundle retainers (second holding members) 134 on the other side of the first and second support portions 106, 108. Moreover, the second tube 82b is disposed in C-ring portions 134a of the bundle retainers (third holding members) 134 in the center between the bundle retainers (first holding members) 134 on one side of the first and second support portions 106, 108 and the bundle retainers (second holding members) 134 on the other side thereof.

The effects and advantages of the medical device 10 according to this embodiment are the same as the effects and advantages of the medical device 10 described in the first embodiment, and are therefore omitting a description thereof.

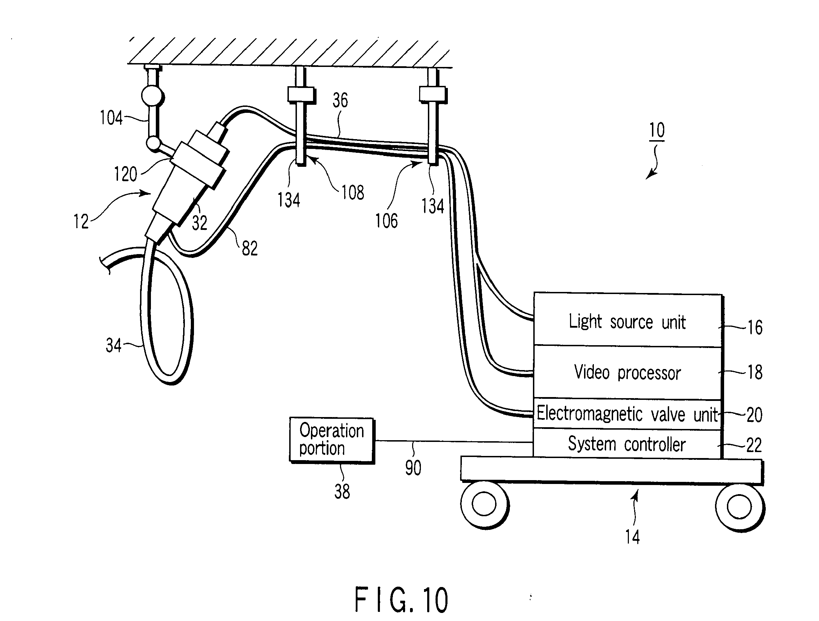

In addition, while the first and second support portions 106, 108 are fixed to the fourth arm 104d here in the above explanation, it is also preferable that the first and second support portions 106, 108 are suspended from the ceiling, as shown in FIG. 10. Moreover, it is also preferable that an arm 104 having the endoscope holding portion 120 at its end is suspended from the ceiling.

Furthermore, while the first to fourth support portions 106, 108, 112, 114 are arranged to extend above or under the arm 104 in the first to forth embodiments described above, it is also preferable that the first to fourth support portions 106, 108, 112, 114 are arranged to extend beside the arm 104.

Still further, the tables 130 of the first to fourth support portions 106, 108, 112, 114 turn about the axis of the shaft 124 or about the axes of the pivot members 138 in the above explanation, it is also preferable that the flange portion 124b can also turn about the axis of the shaft 124.

Further yet, each of the universal cable 36 and the first and second tubes 82a, 82b is supported at two front and rear places by, for example, the first and second support portions 106, 108 in the above explanation, but is also preferably supported at, for example, three places.

Further yet, while the tube 82 has the first tube 82a for air supply/water supply and the second tube 82b for suction in the described embodiment, the lumen 84a for air supply and the lumen 84b for water supply provided in the first tube 82a for air supply/water supply may be independent of each other. In this case, the lumen 84a for air supply, the lumen 84b for water supply and the tube 82b for suction can be independently supported by three bundle retainers 134 of the first support portion 106, as shown in FIG. 9C. Meanwhile, the universal cable 36 can be independently supported by one bundle retainer 134 of the first support portion 106 shown in FIGS. 5A and 5B.

Moreover, while the endoscope 12 is used as a medical instrument in the first to forth embodiments described above, it is possible to use, for example, an electric scalpel in the same manner.

While some of the embodiments have been specifically described above with reference to the drawings, this invention is not limited to the embodiments described above, and covers all the embodiments carried out without departing from the spirit thereof.

### Industrial Applicability

According to this invention, it is possible to provide a medical device capable of maintaining a condition where a medical instrument having a plurality of flexible members extending in different directions or extending from different positions is easily turned or rotated about the axes of the flexible members.

## Claims

1. A medical device (10) **characterized by** comprising:
a medical instrument main body (32);
an elongated and flexible first flexible member (36) extending from the medical instrument main body;
an elongated and flexible second flexible member (82) extending from the medical instrument main body in a direction different from that of the first flexible member or from a different position;
an arm (104) which turnably supports the medical instrument main body;
a first holding member (134) which movably holds the first flexible member; and
a second holding member (134) which movably holds the second flexible member.

2. The medical device (10) according to claim 1,
**characterized in that**
the first holding member (134) includes a holding portion (134a) which holds the first flexible member (36) movably back and forth in its axial direction, and
the second holding member (134) includes a holding portion (134a) which is separated from the first holding member at a predetermined distance and which holds the second flexible member (82) movably back and forth in its axial direction.

3. The medical device (10) according to claim 1,
**characterized in that**
the first holding member (134) includes a turning holding portion (126, 130, 134a) which holds the first flexible member (36) turnably about its axis, and
the second holding member (134) includes a turning holding portion (126, 130, 134a) which is separated from the first holding member at a predetermined distance and which holds the second flexible member (82) turnably about its axis.

4. The medical device (10) according to claim 3,
**characterized in that**
the first holding member (134) includes a holding portion (134a) which holds the first flexible member (36) movably back and forth in its axial direction,
the second holding member (134) includes a holding portion (134a) which holds the second flexible member (82) movably back and forth in its axial direction, and
the first holding member and the second holding member are supported by support portions (106, 108; 112, 114) which support the first flexible member and the second flexible member turnably about their axes.

5. The medical device (10) according to any one of claims 1 to 4, **characterized in that** at least one of the first holding member (134) and the second holding member (134) is provided in the arm (104).

6. The medical device (10) according to any one of claims 1 to 5, **characterized in that** at least one of the first holding member (134) and the second holding member (134) is suspended from a ceiling.

7. The medical device (10) according to any one of claims 1 to 6, **characterized in that** the holding member (134) includes a C-ring (134a) having an opening (134b) through which the first and second flexible members (36, 82) are inserted and removed.

8. A medical device (10) **characterized by** comprising:
an arm (104);
a medical instrument main body (32) turnably supported by the arm;
an elongated and flexible first flexible member (36) extending from the medical instrument main body;
an elongated and flexible second flexible member (82) extending from the medical instrument main body in a direction different from that of the first flexible member or from a different position; and
at least one support portion (106, 108; 112, 114) which movably supports the first flexible member and the second flexible member.

9. The medical device (10) according to claim 8,
**characterized in that**
the support portion (106, 108) includes:
a shaft (124) having one end and the other end and provided with a rotation axis (O₁) at the one end;
a table (130) provided at the one end of the shaft and turnable about the rotation axis; and
at least one holding member (134) fixed to the table.

10. The medical device (10) according to claim 8,
**characterized in that**
the support portion (112, 114) includes:
a shaft (124) having one end and the other end;
a table (130) disposed at the one end of the shaft;
at least one rotation axis (O₂, O₃, O₄) disposed in the table and provided in parallel with the longitudinal direction of the shaft; and
a holding member (134) fixed to the rotation axis and rotatable with respect to the table.

11. The medical device (10) according to claim 8,
**characterized in that**
the support portion (108, 110; 112, 114) includes:
a shaft (124) having one end and the other end and provided with a first rotation axis (O₁) at the one end;
a table (130) disposed at the one end of the shaft and turnable about the first rotation axis;
a plurality of second rotation axes (O₂, O₃, O₄) provided in the table; and
a holding member (134) fixed to the second rotation axes and rotatable with respect to the table.

12. The medical device (10) according to any one of claims 9 to 11, **characterized in that** the holding member (134) includes a C-ring (134a) having an opening (134b).

13. The medical device (10) according to any one of claims 8 to 12, **characterized in that** the other end of the shaft (124) is supported by the arm (104).

14. The medical device (10) according to any one of claims 8 to 12, **characterized in that** the other end of the shaft (124) is suspended from a ceiling.
